# EUROPEAN PATENT SPECIFICATION

(11) **EP 0 505 478 B1**
(45) Date of publication and mention of the grant of the patent: **27.11.1996**
(21) Application number: 91902037.0
(22) Date of filing: 14.12.1990
(51) Int. Cl.: A61M 35/00

(54) **A TREATMENT SYSTEM FOR WOUNDS AND OTHER DISORDERS**
BEHANDLUNGSSYSTEM FÜR WUNDEN UND ANDERE FEHLER
SYSTEME DE TRAITEMENT DE PLAIES AINSI QUE D'AUTRES AFFECTIONS

(30) Priority: 14.12.1989 US 451957
(43) Date of publication of application: 30.09.1992
(73) Proprietor: Eriksson, Elof, Boston, MA 02115 (US)
(72) Inventor: Eriksson, Elof, Boston, MA 02115 (US)
(74) Representative: Bassett, Richard Simon
(86) International application number: US9007469
(87) International publication number: WO9108793

(56) References cited:
- EP-A- 0 035 583
- DE-A- 2 544 884
- DE-C- 2 628 719
- FR-A- 2 252 839
- FR-A- 2 390 177
- US-A- 3 026 874
- US-A- 3 367 332
- US-A- 4 375 812
- US-A- 4 382 441

## Description

The invention relates to a system for diagnosis and treatment of wounds and other skin disorders. More particularly, the invention relates to a treatment system for wounds, burns, and skin diseases, to enhance the rate of healing and decrease scarring.

Most open wounds are currently treated with moist or dry gauze. This results in excessive pain, dehydration of the wound, loss of fluids and proteins, loss of heat and delayed healing. Patients are also treated topically and systemically with antibiotics to prevent or control infection.

Open wounds appear to heal faster in an environment which is somewhere between moist and dry. Partial thickness wounds heal faster when covered with a polyethylene film than when exposed to air. Conventionally, dressings with some water permeability provide the optimal conditions for healing.

The concept of protecting a wound from the environment and using fluid to treat the wound is generally known, but has not been implemented clinically. For example, treating wounds by fluid treatment is disclosed in United States Patent Nos. 3,089,429 to Owens, 3,367,323 to Groves, 4,375,812 to Vaseen and 4,382,441 to Svedman. Fluid treatment has been recognized as useful in cleansing wounds and removing foreign particles in a "stream" fluid treatment system, for example, as described in United States Patent No. 3,288,140 to McCarthy. Groves utilizes a semi-permeable membrane as part of the bandage (chamber) to allow passage of some matter out of the enclosed environment and Svedman indicates that the system described therein acts to remove degradation products. The system described in Svedman also acts as a transport system for supplying the tissue with nutritive substances.

Apparatus used in connection with fluid treatment systems where wounds are enclosed in a chamber allowing the administration of antibiotics and other medications are also known in the art. For example, the apparatus disclosed by United States Patent No. 3,026,874 issued to Stevens includes a chamber, a seal between the chamber and the skin, a transparent "window", and an inlet and an outlet port. Groves and Svedman disclose variations on this concept.

FR-A-2252839 discloses a bag with an elastic cuff which grips the wrist when a hand is introduced into the bag. The apparatus is used to treat wounds with oxygen.

However, none of the systems described above have gained clinical acceptance. These apparatus and methods fail to provide the capability to diagnose the condition of the wound. Further, most of the prior art systems were designed for the use of a single treatment modality or single drug treatment which greatly limits their usefulness. None lead to an enhanced rate of wound healing or comprehensive control over the wound healing process.

It is therefore an object of the present invention to provide an apparatus and treatment fluid, and methods of use thereof, leading to enhanced healing and decreased scarring of superficial wounds and diseases.

It is a further object of the present invention to provide such apparatus and methods that provide for continuous, discontinuous, and readily modifiable treatment of wounds and diseases.

### Summary of the Invention

One aspect of the invention provides a treatment system for wounds, said treatment system comprising: a flexible, conformable chamber having a bellows fold for containing treatment fluid therein and sealable about the periphery of a wound; treatment fluid comprising saline and containing oxygen, carbon dioxide, and a physiological pH suitable for wound healing within the chamber; and monitoring means for assessing wound conditions, wherein the chamber allows control over oxygen concentration, carbon dioxide, and pH.

The system optionally includes portal means for introduction and removal of treatment fluids from said chamber, control means for treatment variables, and monitoring means for monitoring wound conditions, in combination with a treatment fluid consisting of saline and other optional additives such as antibiotics and anesthetics. Treatment additives are selected according to wound indications and include growth factors, antibiotics, anesthetics, enzymes and oxygen. Treatment variables, such as temperature, colloid osmotic pressure, pH, ion concentration and oxygen content, are controlled according to wound indications. The treatment fluid is selected according to wound indications and may be changed periodically according to wound indications or continuously perfused through the chamber.

Monitoring is accomplished by visual inspection of the wound and the treatment fluid through a transparent section of the chamber and/or by extracting fluid from the system and analyzing for protein content and microorganisms.

In some embodiments, the chamber includes a peripheral adhesive surface, and a portion of the chamber is self repairing. The chamber can also include a releasable compartment.

In a variation of the method for treating wounds, the treatment system is used for dialysis.

### Brief Description of the Drawings

Figure 1 is an illustration of a preferred embodiment of a chamber according to the invention; Figure 1a is a prospective view; Figure 1b is a cross-sectional view;
Figure 2 is an illustration of another preferred embodiment of a chamber according to the invention;
Figure 3 is a graph of volume of chamber fluid (ml) above burn wounds over time (days after burning) (Fluid measured and replaced (5 ml) daily; Means with 95% confidence intervals, numbers of replicates in parentheses. There was no measurable fluid flux across unburned skin.);
Figure 4 is a graph of burn protein and electrolyte efflux (mg/cm²/day from a typical burn) (Protein, K and Ca collected daily in saline-filled chambers above a typical burn wound);
Figure 5 is a graph of protein efflux from four burns (mg/cm²/day) (protein collected daily in saline-filled chambers above single burns in four different pigs. Horizontal bar at bottom represents efflux through unburned skin);
Figure 6 is a graph of influence of growth factors on burn wound healing times (mean and 95% Cl in six pigs; p < 0.05);
Figure 7 is a graph of influence of growth factors on excisional wound healing times (mean and 95% Cl in four pigs).

### Detailed Description of the Invention

The treatment system and method of use thereof recreates the physiological environment at a wound site, while providing protection from the environment, thereby decreasing susceptibility to infection and enhancing wound healing while minimizing scarring.

The term "wound" as used herein refers to a number of conditions including, but not limited to, burn wounds, incisional wounds, excisional wounds, tumors, skin diseases and other skin or superficial disorders. The term "enhanced wound healing" as used herein means an accelerated rate of healing, better quality of healing (i.e., healing with less scarring), healing of wounds that would otherwise not heal and the improvement and elimination of other skin conditions. Other skin conditions include conditions normally referred to as cosmetic, such as wrinkles.

Since the system primarily administers treatment agents topically, rather than systemically, greater concentrations and utilization of otherwise toxic materials, can be used.

### The treatment apparatus

The treatment system generally includes a chamber in combination with a treatment fluid such as saline, and optionally includes treatment additives such as antibiotics, anesthetics, growth factors, or even skin cell dispersions for grafting to burn wounds, and means for control over treatment variables and for monitoring of wound conditions. The chamber encloses a predetermined surface area about the wound. The chamber provides protection for the wound from the surrounding non-sterile environment, control of treatment variables, containment for continuous fluid treatment, an effective delivery system, an effective interface between the wound and the environment, a direct monitoring of wound physiology and wound diagnosis as well as an exchange of ions and waste materials by movement through a concentration gradient (dialysis). Another aspect of the treatment system is the use of the treatment system to control pain, which can be accomplished through intact skin, as well as at wound sites.

In the preferred embodiment, wound conditions are regularly monitored. The ongoing presence of protein and microorganisms in the treatment fluid is indicative of a need for a change in the treatment protocol. This fluid can be extracted by means of a syringe through a self-repairing section of the flexible chamber wall or by continuous or discontinuous extraction of fluid from the chamber through inlet and outlet ports. A transparent section of the chamber can also be used for direct visual monitoring of the wound conditions.

Valve mechanisms are necessary where the apparatus is not to be connected to a treatment fluid reservoir and a drain or connected to a continuous perfusion system. However, in some cases, such as an emergency care version of the system, the ports may be sealed. The apparatus would initially be applied at an accident site and an inner compartment containing treatment fluid opened allowing treatment fluid to enter the chamber. The seals of the ports would be broken at an appropriate time for connection to other apparatus, such as a continuous perfusion system, at a hospital, for example.

A preferred embodiment of the treatment system incorporates continuous perfusion of treatment fluid through inlet and outlet ports. A pump or gravity may be used to move the treatment fluid. The treatment fluid may be recirculated after filtering and other appropriate action (eg. heating or cooling). Alternately, fresh treatment fluid may be introduced and contaminated fluid disposed of.

As noted above, there are a number of treatment variables which may be controlled by the system. One such treatment variable which may be controlled is temperature. It has been found that heating the wound from a temperature of approximately 27°C (a common temperature of a lower extremity wound) to 37°C accelerates wound healing. Experimental data has shown that at a wound temperature of approximately 37°C, the rate of wound healing is more than twice as fast as at a temperature of 27°C. The temperature of the wound area can be increased by heating the treatment fluid. Cooling has also been proven beneficial in the case of acute burn and other traumatic wounds. Cooling reduces pain, swelling and destruction of tissue. In general terms, acute wounds benefit from cooling during the first hours after occurrence of the wound and later, wounds benefit from a temperature of approximately 37°C. Cooling can similarly be effected by cooling the treatment fluid. Generally, a temperature of 20°C is recommended. In acute traumatic wounds and in burn wounds, a temperature of five degrees centigrade is suggested if the wound area is smaller than ten by ten centimeters. In larger wounds, a cooling temperature of 20°C is suggested with monitoring of core temperature.

Fluid extracted from the system can be analyzed for factors which provide an indication of the status of wound healing, including not only microorganisms, but also low oxygen, high carbon dioxide and adverse pH. The fluid may be tested for the number and type of bacteria and other microorganisms, the number and type of host cells, and the amount and type of proteins.

The fluid can also be tested for growth factors that are produced by the wound, as well as the presence and the amounts of various inflammatory mediators and antigens. The presence of antigens can be detected by delivery of specific antibodies through the wound chamber. It is important to note that the chamber establishes an environment that allows the positive factors produced by the body to remain at high concentrations at the wound site.

The treatment system allows control over treatment variables including temperature, specific ion concentration, colloid osmotic pressure, glucose concentration, amino acid content, fat concentration, oxygen concentration and carbon dioxide concentration and pH. Control over these additional treatment variables can significantly enhance wound healing.

Another advantage of this system is that the treatment fluid can be applied sequentially, alone or in combination with various treatment additives and with changes in treatment variables. The sequential application may be predetermined or it may be responsive to monitoring. In the preferred embodiment, the chamber for enclosing a predetermined surface area about the wound is sealed to the skin surface about the wound by means of an adhesive. An appropriate treatment fluid is introduced into the sealed chamber. The treatment fluid may be introduced and then extracted in favor of fresh treatment fluid or treatment fluid may be pumped through the chamber. Selected treatment additives may be introduced into the chamber continuously or at a predetermined time or at periodic intervals. Appropriate control of treatment variables is also effected. Monitoring is accomplished by examination of the patient and visual examination of the fluid within the chamber and the wound itself. In addition, samples of fluid are extracted from the chamber for analysis and diagnosis. Intervention and further treatment is based upon the monitoring data. The chamber is removed once sufficient healing of the wound is diagnosed.

One method for determining whether or not the wound is healed involves measuring the protein content of the extracted fluid. When the protein content of the extracted fluid decreases to baseline, the wound is healed. Methods for determining protein content are well known in the art and are inexpensive and fast. It is also contemplated that the types of protein and the relative amounts of the types of protein may provide further data for monitoring. It is important to note that analyzing the extracted fluid is a non-invasive means of monitoring the healing process as well as a more precise means.

The system can be used in a variety of applications. As a general drug delivery system, it could be used not only in hospital and outpatient medical settings, but also by individual patients, for emergency care or long term care. It can be pre-packaged for extended storage, rough handling and simple and quick application. Generally, the system would be sterilely packed.

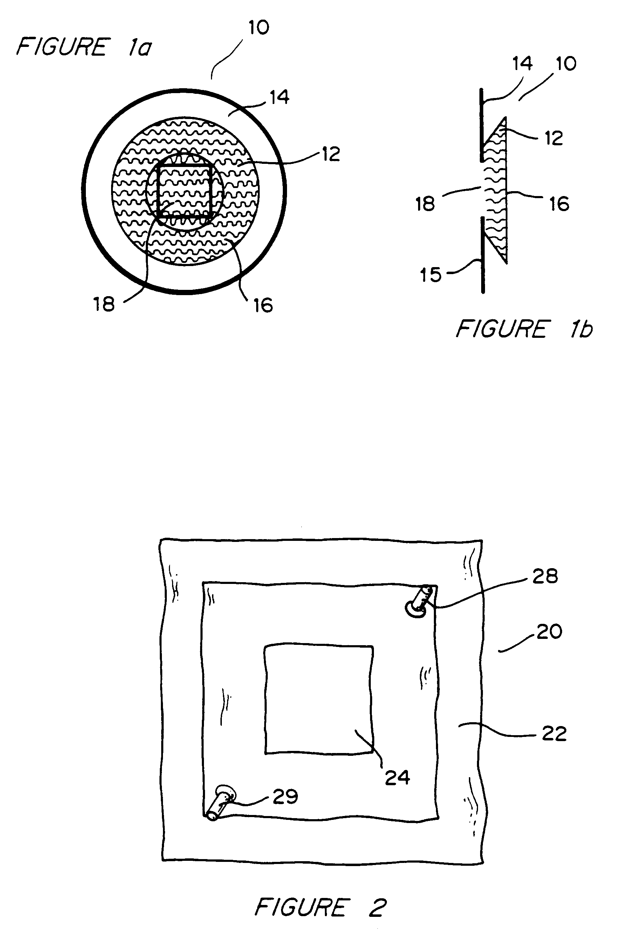

A preferred embodiment of a chamber 10 employed in the examples is shown in Figure 1a and 1b. The chamber 10 is essentially a bandage having a bellows-type construction. The chamber 10 is constructed of a clear, flexible, biocompatible plastic, such as vinyl, which is also non-reactive with the treatment fluid and additives. The bellows fold 12 allows for a large capacity for treatment fluid as well as ease of construction. The chamber 10 can be made by welding an annular base 14 by heat or ultrasound to an annular bellows fold 12 piece and welding a circular top 16 to bellows fold 12 piece. Circular top 16 is a substantially transparent material. The remaining components are not necessarily transparent but may be. A transparent annular base can be used to provide for easy visual inspection for leakage. An opening 18 in the annular base 14 is provided, allowing the treatment fluid access to the wound. The bottom side 15 of annular base 14 is provided with an adhesive tape or coating suitable for securing the chamber to skin. As those skilled in the art will readily recognize, a removable sheet for protecting the adhesive and maintaining the sterility of the interior of the chamber is desirable. The chambers may be stored in a sterile pack for years. This chamber can take many shapes in order to fit wounds from the size of one square centimeter up to the size of a whole extremity. It is important that the adhesive surface be sufficient to secure the bandage to the skin surface to ensure a leak-proof seal.

To provide a means for introducing treatment fluid into the chamber 10, the chamber can be made of a self-repairing material. Alternatively, inlet and outlet ports allowing the introduction and extraction of various substances into the chamber can be provided.

A multi-compartmental chamber can also be used. The chamber includes a release mechanism allowing the treatment fluid to enter the main part of the chamber after the chamber is secured to the patient. Further compartments within the chamber containing treatment additives may be provided. A compartment which contains a chemical or electrochemical heating or cooling system may also be provided to effect treatment variable control. Prepackaging of a system is especially useful for emergency care applications as well as over-the-counter versions. A skin-cleaning set may be provided together with such a system in order to ensure a leak-free seal between the chamber and the skin. An outer covering for "field" protection would also be available.

The shelf-life of a prepackaged system is limited only by the selection of treatment fluid and treatment additives. The chamber and treatment additives such as pain medication and antibiotics are stable for years if provided unmixed in a dry compartment together with a separate compartment for a treatment fluid such as saline.

The system also has application as a general drug delivery system. Skin permeability increases with hydration. Accordingly, various drugs can be administered without ingestion or injection. Small molecule drugs such as local anesthetics, cortisone, morphine, insulin, and antibiotics are particularly suitable for delivery using this apparatus. A chamber, such as the one illustrated in Figure 1 or Figure 2 is secured to the skin of the patient in a suitable location. Treatment fluid and the drug to be administered is then introduced into the chamber. The amount of time to hydrate the skin may be increased or an enzyme which destroys the outer epidermal layer may be included in the treatment fluid to increase the rate of penetration.

In another embodiment particularly useful in prepackaged versions of the system, a permeable or semi-permeable container may be formed as part of the chamber as a compartment. The interior wall of the compartment is formed from a permeable or semi-permeable material. A suitable non-permeable cover which can be removed before application of the chamber or released after application of the chamber may be employed to protect the membrane and the contents of the compartment. The outer wall of the compartment may be fitted with a portal such as an inlet port so that additional treatment additive may be added.

A preferred embodiment of the apparatus is used for delivery of local anesthesia to intact skin. A pre-packaged system having a bellows bandage type chamber 20 and a releasable compartment containing 2-6 ccs of lidocaine 5% as the treatment fluid is shown in Figure 2. The efficacy of lidocaine in anesthetizing intact skin has been documented. However, a practical delivery system has previously been lacking. The chamber is applied to the area designated for injection approximately four hours before injection and the treatment fluid allowed to enter the chamber from the releasable compartment. Alternatively, a 1% solution of dyclonine is especially effective as a local anesthetic after only twenty to thirty minutes. Benzocaine, in about a 20% solution, is also useful as a local anesthetic treatment additive.

In another embodiment of the invention, a time release system is provided. Treatment additives with or without a suitable carrier may take the form of pellets or other similar packaging that allows the treatment additive to be dissolved into the treatment fluid over time. Thus, a treatment additive pellet may be introduced into a chamber containing treatment fluid where it will dissolve over time, thereby increasing treatment additive concentration or replacing depleted treatment additive. Instead of forming treatment additive or treatment additive with a carrier into a pellet form, the treatment additive may be placed into a permeable or semi-permeable container. The container then allows treatment additive to dissolve into the treatment fluid. Selection of the permeable material may be made such that treatment additive only enters the treatment fluid when the balance of treatment additive in the treatment fluid falls below a certain concentration or when some other factor, such as temperature or pH, acts on the container to allow treatment additive to dissolve into the treatment fluid. A natural feedback system for introducing the desired treatment additive is thereby created.

Control over pH may be effectively achieved in this matter. Buffering of the fluid in the treatment system is important. Other approaches to this problem include making the quantity of treatment fluid so large so as to minimize the effect or so small that the wound fluid can provide the buffer.

Another preferred embodiment of a chamber 20 according to the invention is shown in Figure 2. Figure 2 shows chamber 20 secured to a limb 26. Chamber 20 is constructed of vinyl or other suitable material. An adhesive periphery 22 is provided and the central portion 24 is substantially transparent. There is an inlet port 28 and outlet port 29. Some chambers may be provided only with a single port for both inlet and outlet, or the port may only allow the introduction of substances via a one-way valve. Extraction of fluid may, in such a case, be accomplished with a syringe or at such time as the chamber is removed. It is further contemplated that separate fittings for ports may be attached to a chamber before or during the time the chamber is secured to the patient.

Fittings appropriate for inlet and outlet ports are shaped and configured for connection to tubing that may be part of a fluid introduction or extraction system, including a continuous perfusion system.

A plurality of chambers can be used to form a treatment system body suit. Chambers may be formed in a variety of patterns and configurations to cover a body or part of a body. H-type joint tape can be used to join adjoining chambers and inlet or outlet ports provided at various convenient locations.

Before applying a chamber to a patient, the skin about the wound is first cleaned to remove materials that might interfere with adhesion and disinfected. For example, the skin can be scrubbed with Betadine soap, dried and Betadine solution applied. In the event that the chamber does not include an adhesive material, a medical liquid adhesive (acrylic, Hollister) can be painted onto the area surrounding the wound where the device is going to be attached. The backing of the adhesive surface of the device is removed and the device is applied over the wound by pressing on the adhesive surface.

### Treatment fluid and additives.

Normal saline is the basic treatment fluid. Buffers may also be part of the basic treatment fluid. For emergency use, it may be kept refrigerated in order to provide cooling of the burn wound. However, the treatment fluid generally does not require refrigeration. A preferred basic treatment fluid includes 0.9% sodium chloride with 0.001% lidocaine (10 micrograms per ml). After initial introduction into the treatment system, the treatment fluid is left in place for 2 to 24 hours, depending on wound type.

### Antimicrobials.

Basic treatment fluid may also include antimicrobials for the treatment of bacterial, fungi or viral infections. Penicillin at 100 IU/ml and Streptomycin at 100 µg/ml provides appropriate concentrations for a general antibiotic component of the treatment fluid.

### Anesthetics.

Local anesthetics such as lidocaine can be utilized for localized pain control. Allergies to lidocaine are almost nonexistent.

### Chemotherapeutics.

Chemotherapeutics can also be directly added to the treatment fluid for treatment of tumors or other cancers. There are many chemotherapeutic agents available that can be used that are approved by the Food and Drug Administration, and may be drugs or proteins such as specific monoclonal antibodies to tumor antigens.

### Growth Factors.

The following growth factors have been studied in experimental pig burn wound and excisional wound models as treatment additives: epidermal growth factor (EGF), platelet-derived growth factor (PDGF), insulin-like growth factor (IGF), basic fibroblast growth factor (bFGF), and cholera toxin (CT). Growth factors can also be produced by the wound itself.

### Immunomodulators.

Agents that suppress or enhance the immune response can also be added. These may be immunosuppressants such as cyclosporin or cortisone, or immunostimulants or modifiers. In other skin conditions such as eczema, treatment additives such as cortisone and other immune modulators are added to the treatment fluid to suppress the immune reaction.

Immunostimulants may be used to attract phagocytic cells to the wound to enhance clearing of necrotic tissue and microorganisms. Proteases may also be added to promote removal of necrotic tissue.

### Tissue culture mediums and osmotic agents.

Tissue culture mediums for enhancing growth of cells, alone or in combination with dispersed cells, and fluids which increase osmotic pressure and oxygen accessibility may also be introduced into the chamber as treatment additives, for example, dextran or glucose.

If the wound is of full thickness without any skin elements in the wound itself, autogenous keratinocytes can be harvested from the lower abdomen, minced, trypsinized and injected into the chamber. The dispersed cells will deposit and grow in the wound, forming normal stratified skin. Other cell types can also be grafted into the wound using similar technology. In most cases, ion concentrations should be kept to normal extracellular concentrations. Colloid osmotic pressure should be maintained at a high level, about 1.5 to 2 times that of plasma, in order to prevent edema formulation. The colloid osmotic pressure of the treatment fluid is generally not adjusted in wounds smaller than ten by ten centimeters. In larger wounds, osmotic pressure is controlled to two times the osmotic pressure of plasma using a Dextran 40 solution.

Glucose, amino acid and fat concentrations should be kept close to that of plasma or corresponding to a skin tissue culture medium. Furthermore, oxygen and carbon dioxide concentrations should be maintained at their normal tissue levels. Oxygen is an effective treatment additive. The addition of oxygen in the chamber is useful in hypoxic wounds.

Treatment additives are wound specific. For example, if an infection has been diagnosed, antibiotics are added in the amount of one single parenteral dose per 1,000 cc of fluid. Addition of gentamicin, tobramycin or carbenicillin is appropriate for a wound infection with *Pseudomonas*. When hypoxia has been diagnosed, the liquid is passed through an oxygenating chamber before entering the chamber. If a tumor has been diagnosed, chemotherapy is given in an amount of one single parenteral dose per 1,000 cc of fluid. In situations involving a wound containing necrotic tissue and debris, proteolytic enzyme is added to the liquid. Immunomodulators are added to the treatment fluid if an inflammatory reaction is present or desired (inflammatory reactions are essential to healing and removal of debris). Epidermal growth factor is added in a concentration of 10 nanograms per cc when required.

When it has been determined that surgical transplantation of skin is necessary, the treatment system only serves as 24 hours of pretreatment for the skin grafting procedure. In instances of large exposed skin areas not yet ready for skin grafting, the treatment system is left in place until such a procedure can take place. If the wound was extensively infected at the time treatment was initiated, samples for analyses of number and type of bacteria, should be taken each time the chamber fluid is exchanged.

Indication Specific Charts 1-11 are representative of the various wounds for which the present system can be used to enhance healing. In the left most column of each indication specific chart is listed the wound type. From left to right the columns provide suggested treatment fluid, treatment fluid additives and control of treatment variables.

| **CHART 1 - PLASTIC SURGERY** | | | | | |
|---|---|---|---|---|---|
| WOUND TYPE | TREATMENT FLUID | TREATMENT ADDITIVES | FLUID CHANGE | ADDITIVE ADJUSTMENT | MONITOR & ANALYSIS |
| Incisional | Normal Saline with Penicillin & Streptomycin | Immune Modulators Lidocaine Specific Antibiotics | q24h | q24h | Visual Inspection |
| Burn | Normal Saline with Penicillin & Streptomycin | Immune Modulators Lidocaine Specific Antibiotics | 2 hrs x 24h | 2 | Protein Microorganisms |
| Traumatic | Normal Saline with Penicillin & Streptomycin | Immune Modulators Lidocaine Specific Antibiotics | q6h | 6 | Protein Microorganisms |
| Infected | Normal Saline with Penicillin & Streptomycin | Immune Modulators Lidocaine Specific Antibiotics | q2h | 12 | Protein Microorganisms |
| Diabetic | Normal Saline with Penicillin & Streptomycin | Immune Modulators Lidocaine Specific Antibiotics | q12h | 12 | Protein Microorganisms |
| Vascular | Normal Saline with Penicillin & Streptomycin | Immune Modulators Lidocaine Specific Antibiotics | q12h | q12h | Protein Microorganisms |
| Pressure Sores | Normal Saline with Penicillin & Streptomycin | Immune Modulators Lidocaine Specific Antibiotics | q12h | 12 | Protein Microorganisms |

| **CHART 2 - ORTHOPEDIC SURGERY** | | | | | |
|---|---|---|---|---|---|
| WOUND TYPE | TREATMENT FLUID | TREATMENT ADDITIVES | FLUID CHANGE | ADDITIVE ADJUSTMENT | MONITOR & ANALYSIS |
| Open Fractures | Normal Saline with Penicillin & Streptomycin | Lidocaine Growth Factors Specific Antibiotics | q12h | q12h | Protein Microorganisms |
| Open Joints | Normal Saline with Penicillin & Streptomycin | Lidocaine Growth Factors Specific Antibiotics | q12h | 12 | Protein Microorganisms |
| Exposed Prosthetic Material | Normal Saline with Penicillin & Streptomycin | Lidocaine Growth Factors Specific Antibiotics | q12h | 12 | Protein Microorganisms |
| Osteo-Myelitis | Normal Saline with Penicillin & Streptomycin | Lidocaine Growth Factors Specific Antibiotics | q12h | 12 | Protein Microorganisms |
| Traumatic | Normal Saline with Penicillin & Streptomycin | Lidocaine Growth Factors Specific Antibiotics | q12h | 12 | Protein Microorganisms |
| Incisional | Normal Saline with Penicillin & Streptomycin | Lidocaine Growth Factors Specific Antibiotics | q12h | q12h | Protein Microorganisms |

| **CHART 3 - DERMATOLOGY** | | | | | |
|---|---|---|---|---|---|
| WOUND TYPE | TREATMENT FLUID | TREATMENT ADDITIVES | FLUID CHANGE | ADDITIVE ADJUSTMENT | MONITOR & ANALYSIS |
| Psoriasis | Normal Saline with Penicillin & Streptomycin | Hydrocortisone (1 mg/cc) Non-steroidal anti-inflammatory agents | q12h | q12h | Visual Inspection |
| Acne | Normal Saline with Penicillin & Streptomycin | Clindomycin | q12h | 12 | Microorganisms |
| Bacterial | Normal Saline with Penicillin & Streptomycin | Specific Antibiotics | q6h | 6 | Microorganisms |
| Viral Infection | Normal Saline with Penicillin & Streptomycin | Acyclovir | q12h | 12 | Antibodies Microorganisms |
| Benign Neoplasms | Normal Saline with Penicillin & Streptomycin | Keratolytic Agents (such as salicylic acid) | q24h | 24 | Visual Inspection |
| Malignant Neoplasms | Normal Saline with Penicillin & Streptomycin | Fluorouracil (3 mgs/ml) | q12h | 12 | Protein Tumor Antigens Visual Inspection |
| Mycosis Fungoides | Normal Saline with Penicillin & Streptomycin | Hydrocortisone (3mgs/ml) Bleomycin (1 mg/cc) | q12h | 12 | Protein Microorganisms |

| **CHART 4 - CARDIO-THORACIC SURGERY** | | | | | |
|---|---|---|---|---|---|
| WOUND TYPE | TREATMENT FLUID | TREATMENT ADDITIVES | FLUID CHANGE | ADDITIVE ADJUSTMENT | MONITOR & ANALYSIS |
| Open Chest | Normal Saline with Penicillin & Streptomycin | Lidocaine Growth Factors Specific Antibiotics | q12h | q12h | Protein Microorganisms |
| Open Empyemas | Normal Saline with Penicillin & Streptomycin | Lidocaine Growth Factors Specific Antibiotics | q12h | 12 | Protein Microorganisms |
| Incisional | Normal Saline with Penicillin & Streptomycin | Lidocaine Immune Modulators | q12h | 12 | Visual |

| **CHART 5 - OTO-RHINO-LARYNGOLOGY** | | | | | |
|---|---|---|---|---|---|
| WOUND TYPE | TREATMENT FLUID | TREATMENT ADDITIVES | FLUID CHANGE | ADDITIVE ADJUSTMENT | MONITOR & ANALYSIS |
| Chondritis of the Ear | Normal Saline with Penicillin & Streptomycin | Lidocaine Growth Factors Specific Antibiotics | q12h | q12h | Protein Microorganisms |
| Open Wounds | Normal Saline with Penicillin & Streptomycin | Lidocaine Growth Factors Specific Antibiotics | q12h | 12 | Protein Microorganisms |
| Incisional | Normal Saline with Penicillin & Streptomycin | Lidocaine Immune Modulators | q12h | q12h | Visual |

| **CHART 6 - NEUROSURGERY** | | | | | |
|---|---|---|---|---|---|
| WOUND TYPE | TREATMENT FLUID | TREATMENT ADDITIVES | FLUID CHANGE | ADDITIVE ADJUSTMENT | MONITOR & ANALYSIS |
| Open Wounds | Normal Saline with Penicillin & Streptomycin | Lidocaine Growth Factors Specific Antibiotics | q12h | q12h | Protein Microorganisms |
| Superficial Brain Abcesses | Normal Saline with Penicillin & Streptomycin | Lidocaine Specific Antibiotics Growth Factors | q6h | 6 | Protein Microorganisms |
| Infected | Normal Saline with Penicillin & Streptomycin | Lidocaine Specific Antibiotics Growth Factors | q6h | 6 | Protein Microorganisms |
| Osteo-Myelitis | Normal Saline with Penicillin & Streptomycin | Lidocaine Specific Antibiotics Growth Factors | q6h | 6 | Protein Microorganisms |

| **CHART 7 - OPTHALMOLOGY** | | | | | |
|---|---|---|---|---|---|
| WOUND TYPE | TREATMENT FLUID | TREATMENT ADDITIVES | FLUID CHANGE | ADDITIVE ADJUSTMENT | MONITOR & ANALYSIS |
| Bacterial Infection | Normal Saline with Penicillin & Streptomycin | Lidocaine Growth Factors Specific Antibiotics | q12h | q12h | Protein Microorganisms |
| Viral Infection | Normal Saline with Penicillin & Streptomycin | Lidocaine Specific Antibiotics Growth Factors | q12h | 12 | Protein Microorganisms |
| Chemical | Normal Saline with Penicillin & Streptomycin | Lidocaine Growth Factors Specific Antibiotics | 1 1/2h | 12 | Protein Microorganisms |
| Osteo-Myelitis | Normal Saline with Penicillin & Streptomycin | Lidocaine Specific Antibiotics Growth Factors | q6h | 6 | Protein Microorganisms |

| **CHART 8 - VASCULAR SURGERY** | | | | | |
|---|---|---|---|---|---|
| WOUND TYPE | TREATMENT FLUID | TREATMENT ADDITIVES | FLUID CHANGE | ADDITIVE ADJUSTMENT | MONITOR & ANALYSIS |
| Open | Normal Saline with Penicillin & Streptomycin | Lidocaine Specific Antibiotics Growth Factors | q12h | q12h | Protein Microorganisms |
| Exposed Prosthetic Material | Normal Saline with Penicillin & Streptomycin | Lidocaine Specific Antibiotics Growth Factors | q12h | q12h | Protein Microorganisms |

| **CHART 9 - GENERAL SURGERY** | | | | | |
|---|---|---|---|---|---|
| WOUND TYPE | TREATMENT FLUID | TREATMENT ADDITIVES | FLUID CHANGE | ADDITIVE ADJUSTMENT | MONITOR & ANALYSIS |
| Infected | Normal Saline with Penicillin & Streptomycin | Lidocaine Specific Antibiotics | q6h | 16h | Bacteria Protein |
| Abcesses | Normal Saline with Penicillin & Streptomycin | Lidocaine Specific Antibiotics | q6h | 6 | Bacteria Protein |
| Incisional | Normal Saline with Penicillin & Streptomycin | Lidocaine Specific Antibiotics | q6h | 6 | Bacteria Protein |

| **CHART 10 - GYNECOLOGY** | | | | | |
|---|---|---|---|---|---|
| WOUND TYPE | TREATMENT FLUID | TREATMENT ADDITIVES | FLUID CHANGE | ADDITIVE ADJUSTMENT | MONITOR & ANALYSIS |
| Vaginal Infection | Normal Saline with Penicillin & Streptomycin | Lidocaine Specific Antibiotics Growth Factors | q6h | q6h | Protein Microorganisms |
| Superficial Vulvar Infection | Normal Saline with Penicillin & Streptomycin | Lidocaine Specific Antibiotics Growth Factors | q6h | 6 | Protein Microorganisms |

| **CHART 11 - MEDICAL ONCOLOGY** | | | | | |
|---|---|---|---|---|---|
| WOUND TYPE | TREATMENT FLUID | TREATMENT ADDITIVES | FLUID CHANGE | ADDITIVE ADJUSTMENT | MONITOR & ANALYSIS |
| Superficial Malignant Tumors | Normal Saline with Penicillin & Streptomycin | Lidocaine Chemotherapeutic Agent | q12h | q12h | Protein Microorganisms |
| Antibiotics to Outpatients | Normal Saline with Penicillin & Streptomycin | Specific Antibiotics Chemotherapeutic Agents | q12h | | Protein Microorganisms |

To facilitate understanding these charts, the first, relating to use of the system following plastic surgery, is described in more detail. A treatment system for the indications noted in Indication Specific Chart I - Plastic Surgery may include chambers which are pre-packaged with treatment fluid or treatment fluid and treatment additives may be introduced into the chamber after application to the wound. The selection of the chamber type is wound specific. The preferred treatment fluid is saline with a combination of EGF and PDGF. Possible other treatment additives would include pain medication, antibiotics, and anti-inflammatory agents. For the treatment period of 0 to 24 hours, treatment additives would include a 100 IU/ml dose of penicillin, streptomycin 100 micrograms/ml, and morphine sulphate 1 ng/ml. From 24-120 hours penicillin, streptomycin, and morphine sulphate (EGF-10 ng/ml) IGF-20 ng/ml) would be added along with trypsinized keratinocytes at 37°C. The treatment fluid would be changed every 6 to 12 hours for the first 24 hours, with daily changing thereafter, as required as a result of monitoring.

Control over treatment variables would include continuous cooling to 34°C for the first 24 hours. Monitoring would include analyzing extracted fluid for protein and microorganisms, with samples extracted every 24 hours. When the number of microorganisms is less than 10⁴ per milliliter, infection has been resolved. Protein levels should remain at less than 24 mg/dl/cm².

The present invention will be further understood from the following non-limiting examples.

### Example 1: Treatment of burn wounds on pigs.

Burn damaged skin no longer functions as a protective interface between the body and the environment, leading to fluid loss and infection. The system was tested on pigs weighing about 40 to 45 kg. One square inch burns of partial thickness, and one square inch excisional of partial thickness were created under general anesthesia. One square inch chambers were applied over the wounds. Treatment fluid consisting of normal saline with penicillin and streptomycin was added to the chambers.

Histology revealed less progression of a zone of coagulation in a liquid environment. After four (4) days, an air-exposed wound, in which the loosely attached burned epidermal layer was removed immediately after burning, displayed a zone of necrosis extending down from the surface through about the top third to half of the dermis. The same wound exhibited rather extensive scarring after a period of twelve days upon removal of the crusted surface.

Sections taken through the wound center at about four days showed the epidermis invaded by bacteria that had also undermined the crust. At twelve days, regenerated epithelium covered the surface but included extensive scar tissue containing numerous fibroblasts. Hardly detectable but highly organized collagen bundles were present in residual dermis.

The air-exposed wound were experimentally compared to burn wounds treated with empty or saline-filled chambers. After four days the burn wound developed bacteria-laden debris which covered the crustless surface. Contrastingly, after four days the burn wound covered by a saline-filled chamber displayed no crust, debris or gross signs of wound infection.

Sections taken through the empty-chamber covered burn wound at the four (4) day mark revealed necrosis zones much smaller than that of the untreated burn. Numerous bacteria were present. No bacteria were apparent in a section taken of the saline-treated burn wound and the necrosis zone was very small.

Histology and standardized surface photographs revealed fewer aberrations (scar) in healing when the liquid treatment was used. After a twelve day period the empty-chamber treated burn wound had reepithelialized but was still somewhat inflamed. The saline wound margins could barely be detected at the twelve day mark.

A section taken through the empty-chamber treated burn wound showed nearly normal epidermis, but also hypervascularity and invading bacteria with focal inflammation at twelve days. A similar section through the saline-treated burn wound, showed that, although slightly hyperplastic, the epidermis had a normal architecture and a regular stratum corneum. The subepidermal tissue above the dermis was very thin and contained organized collagen, and only a small number of fibroblasts. The histologic appearance was close to that of normal skin.

Control wounds (those in dry air) healed in over twelve days. Wounds enclosed in chambers with air healed in about twelve days. Wounds enclosed in chambers with liquid healed in less than nine days.

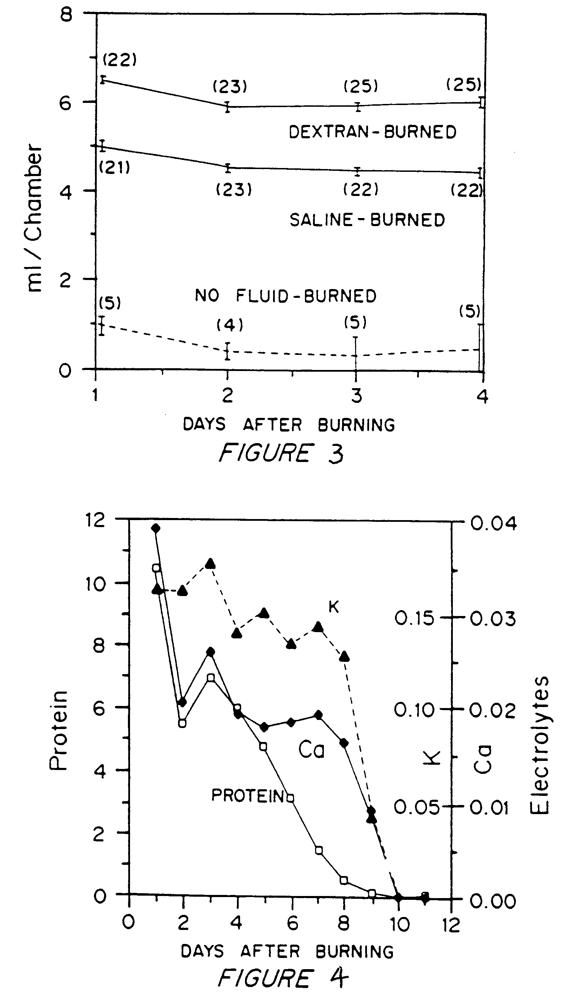

Further tests showed that liquids, proteins and ions move across the wound-liquid interface. The volume of chamber fluid above the burn wounds is shown in Figure 3. There was no measurable fluid flux across unburned skin.

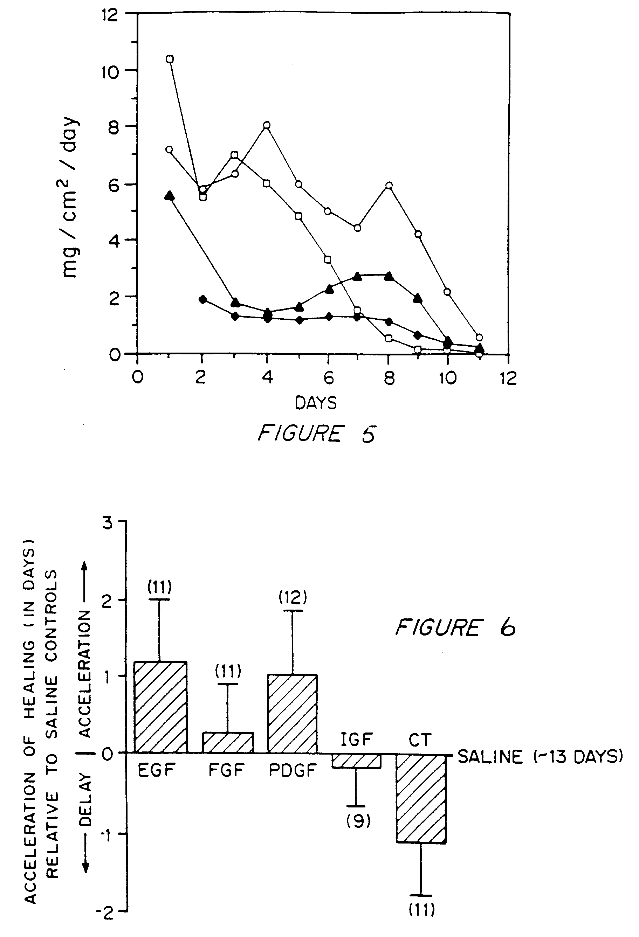

Protein and electrolyte efflux for burn wounds are shown in Figure 4. Protein, K, and Ca were collected daily in Saline-filled chambers above a typical burn wound. Protein leakage coincided with an absence of Surface epithelium, and protein concentrations in the liquid could be used to monitor wound surface epithelium. Figure 5 represents protein collected daily in saline-filled chambers above Single burn wounds in four different subjects. Horizontal bars at the bottom represent the efflux through unburned skin.

### Example 2: Effect of growth factors on healing times of excisional and burn wounds.

The effect of growth factors on the healing times on burn wounds and excisional wounds has also been studied. As shown in Figure 6, in standardized partial thickness burn wounds in pigs, epidermal growth factor (EGF) and platelet-derived growth factor (PDGF) reduce the healing time by approximately one day each. Basic fibroblast growth factor (bFGF) and insulin-like growth factor (IGF) had no effect on the healing times. Cholera toxin (CT) increased the healing times by more than one day.

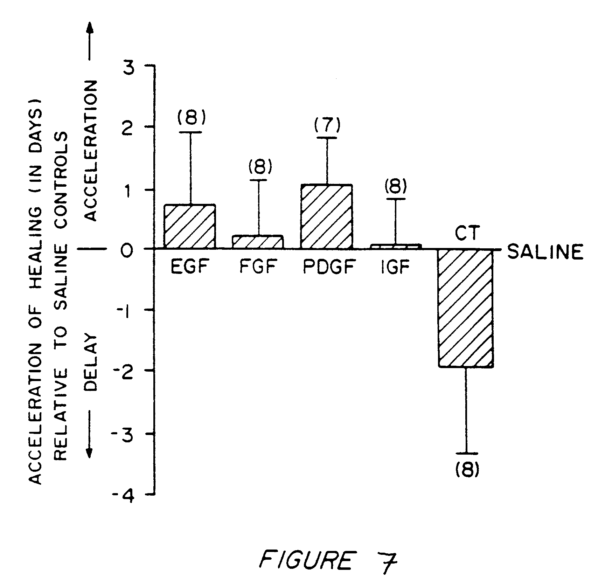

As shown by Figure 7, in partial thickness excisional wounds in pigs, EGF and PDGF reduce healing times while cholera toxin increases the time of healing. bFGF and IGF had no effect.

## Claims

1. A treatment system for wounds, said treatment system comprising:
a flexible, conformable chamber (10, 20, 30, 40, 50') having a bellows fold (12) for containing treatment fluid therein and sealable about the periphery of a wound;
treatment fluid comprising saline and containing oxygen, carbon dioxide, and a physiological pH suitable for wound healing within the chamber; and
monitoring means for assessing wound conditions, wherein the chamber allows control over oxygen concentration, carbon dioxide, and pH.

2. The treatment system according to claim 1 further comprising a treatment additive selected according to wound indications from the group consisting of anesthetics, antibiotics, chemotherapeutics, growth factors, cell culture medium, cells, oxygen, buffering agents, enzymes, and immune modulators.

3. The treatment system according to claim 1 further comprising control means for treatment variables selected from the group consisting of temperature, colloid osmotic pressure, pH, ion concentration, and oxygen content.

4. The treatment system according to claim 2 comprising a treatment fluid selected according to wound indications.

5. The treatment system according to claim 4 further comprising portal means (28, 29, 34, 35) for introduction of treatment fluids into the chamber and for extraction of fluid from said chamber wherein said treatment fluid is changed periodically according to wound indications.

6. The treatment system according to claim 5 comprising means for continuously perfusing treatment fluid through said chamber.

7. The treatment system according to claim 6 wherein said treatment fluid is treated to decontaminate said fluid.

8. The treatment system according to claim 7 comprising means for introducing fresh treatment fluid into the system.

9. The treatment system according to claim 1 wherein said monitoring means comprises visual inspection means whereby the wound may be visually observed and said treatment fluid may be visually observed.

10. The treatment system according to claim 9 wherein said visual inspection means comprises a transparent section (16) of said chamber.

11. The treatment system according to claim 1 wherein said monitoring means comprises means for extracting fluid (28, 29, 34, 35) from the system and means for analyzing said fluid.

12. The treatment system according to claim 11 wherein said means for analyzing said fluid further comprises means for analyzing said extracted fluid for protein content or for microorganisms.

13. The treatment system according to claim 1 wherein said chamber has an adhesive (22) on the surface contacting the periphery of the wound.

14. The treatment system according to claim 1 wherein the chamber comprises a material which is self repairing to allow injection or extraction of material through the chamber walls by a needle.

15. The treatment system according to claim 5 wherein said portal means comprises at least one inlet (28, 34) and outlet port (29, 35).

16. The treatment system according to claim 1 further comprising a second compartment adjacent the fluid-filled chamber having a connection thereto from which a treatment additive can be released into said fluid-filled chamber.

17. The treatment system according to claim 1 wherein said chamber further comprises a compartment within said chamber, said compartment having a permeable membrane.

18. The treatment system according to claim 1 wherein said chamber comprises a:
a first annular ring (14) of flexible sheet material having an inner peripheral edge and an outer peripheral edge and having a top and a bottom;
a second annular ring (12) of flexible sheet material having an inner peripheral edge and an outer peripheral edge, said inner peripheral edge of said second annular ring secured to said inner peripheral edge of said first annular ring; and
a circular piece of flexible sheet material (16) having a peripheral edge secured to said outer edge of said second annular ring;
whereby a collapsible chamber is formed.

19. The treatment system according to claim 18 wherein the bottom of said first annular ring (14) has an adhesive surface.

20. The treatment system according to claim 19 wherein said circular piece of flexible sheet material (16) is substantially transparent.

21. The treatment system according to claim 14 wherein at least a portion of said chamber is self-repairing.

22. The treatment system according to claim 14 wherein said chamber further comprises portal means (28, 29, 34, 35).

23. The treatment system according to claim 22 wherein said portal means comprises at least one inlet (28, 34) and outlet port (29, 35).

24. The treatment system according to claim 18 further comprising a second compartment adjacent the fluid-filled chamber having a connection thereto from which a treatment additive can be released into said fluid-filled chamber.

25. The treatment system according to claim 18 wherein said chamber further comprises a compartment within said chamber, said compartment having a permeable membrane.

26. The treatment system according to claim 1 wherein the periphery of the surface of the chamber contacting the periphery of the wound further comprises an H-type joint tape, said tape comprising parallel planes (42) of flexible sheet material connected by a bridge (44), the outer surfaces of said sheet material having an adhesive surface (43).

27. The treatment system according to claim 1 wherein the fluid-filled chamber further comprises a fastener (52) for joining more than one fluid-filled chamber to each other comprising:
a first attachment strip having an adhesive side and an opposing side secured to a first chamber;
a second attachment strip having an adhesive side and an opposing side secured to a second chamber; and
a bridge having one edge secured to the nonadhesive side of said first attachment strip and the other edge secured to the nonadhesive side of said second attachment strip.

28. The treatment system according to claim 27 wherein said strips and said bridge are a flexible material.

29. The treatment system according to claim 28 wherein said bridge is associated with a stiffening element.

30. The treatment system according to claim 28 wherein said stiffening element is embedded within said bridge.

31. The treatment system according to claim 6 for dialysis wherein said treatment additives and control of said treatment variables are selected to create a favourable gradient.

32. The treatment system according to claim 1 further comprising in the fluid-filled chamber a drug to be delivered to the wound.

33. The treatment system according to claim 2 wherein said treatment additive is analogous skin cells.

34. The treatment system according to any one of claims 1 to 33 for use in medicine.

## Patentansprüche

1. Behandlungssystem für Wunden, umfassend
eine flexible, anpaßbare bzw. konkordante Kammer (10, 20, 30, 40, 50') mit einer Balgenfalte (12) zur Aufnahme eines Behandlungsfluids, die um den Umfang einer Wunde herum verklebbar ist,
ein in der Kammer befindliches Behandlungsfluidum in Form einer sauerstoff- und kohlendioxidhaltigen und einen für die Wundheilung geeigneten physiologischen pH-Wert aufweisenden physiologischen Kochsalzlösung und
ein Überwachungsmittel zur Beurteilung von Wundenzuständen, wobei die Kammer eine Steuerung von Sauerstoffkonzentration, Kohlendioxid und pH-Wert zuläßt.

2. Behandlungssystem nach Anspruch 1, ferner enthaltend einen Behandlungszusatz, entsprechend Wundenindikationen ausgewählt aus der Gruppe Anästhetika, Antibiotika, Chemotherapeutika, Wachstumsfaktoren, Zellkulturmedium, Zellen, Sauerstoff, Puffer(mittel), Enzyme und Immunmodulatoren.

3. Behandlungssystem nach Anspruch 1, ferner enthaltend ein Steuermittel für Behandlungsvariable bzw. -veränderliche, ausgewählt aus der Gruppe Temperatur, Kolloidosmosedruck, pH-Wert, Ionenkonzentration und Sauerstoffgehalt.

4. Behandlungssystem nach Anspruch 2, mit einem entsprechend der Wundenindikation ausgewählten Behandlungsfluidum.

5. Behandlungssystem nach Anspruch 4, ferner enthaltend Zulaßmittel (28, 29, 34, 35) zum Einführen von Behandlungsfluiden in die Kammer und zum Abziehen von Fluidum aus der Kammer, wobei das Behandlungsfluidum entsprechend der Wundenindikation periodisch geändert wird.

6. Behandlungssystem nach Anspruch 5, mit einer Einrichtung zum kontinuierlichen Hindurchströmenlassen von Behandlungsfluidum durch die Kammer.

7. Behandlungssystem nach Anspruch 6, wobei das Behandlungsfluidum einer Dekontaminationsbehandlung unterworfen ist.

8. Behandlungssystem nach Anspruch 7, mit Mitteln zum Einleiten von frischem Behandlungsfluidum in das System.

9. Behandlungssystem nach Anspruch 1, wobei das Überwachungsmittel ein Sichtprüfmittel umfaßt, so daß sowohl die Wunde als auch das Behandlungsfluidum visuell beobachtet oder betrachtet werden kann.

10. Behandlungssystem nach Anspruch 9, wobei das Sichtprüfmittel aus einer transparenten Sektion (16) der Kammer besteht.

11. Behandlungssystem nach Anspruch 1, wobei das Überwachungsmittel (ein) Mittel zum Abziehen von Fluidum (28, 29, 34, 35) aus dem System und ein Mittel zum Analysieren des Fluids umfaßt.

12. Behandlungssystem nach Anspruch 11, wobei das Mittel zum Analysieren des Fluids ferner ein Mittel zum Analysieren des abgezogenen Fluidums auf Proteingehalt oder Mikroorganismen umfaßt.

13. Behandlungssystem nach Anspruch 1, wobei die Kammer an der den Umfang (oder Rand) der Wunde kontaktierenden Fläche ein Klebmittel (22) aufweist.

14. Behandlungssystem nach Anspruch 1, wobei die Kammer ein selbstreparierendes bzw. -schließendes Material zur Ermöglichung des Injizierens oder Abziehens von Material durch die Kammerwände mittels einer Nadel aufweist.

15. Behandlungssystem nach Anspruch 5, wobei das Zulaßmittel mindestens (je) einen Einlaß (28, 34) und eine(n) Auslaß(öffnung) (29, 35) aufweist.

16. Behandlungssystem nach Anspruch 1, ferner umfassend einen neben der fluidumgefüllten Kammer angeordneten und eine Verbindung damit aufweisenden zweiten Raum, aus welchem ein Behandlungszusatz in die fluidumgefüllte Kammer freigesetzt werden kann.

17. Behandlungssystem nach Anspruch 1, wobei ferner innerhalb der Kammer ein mit einer permeablen Membran versehener Raum vorgesehen ist.

18. Behandlungssystem nach Anspruch 1, wobei die Kammer umfaßt:
einen ersten umlaufenden Ring (14) aus flexiblem Folienmaterial mit einem Innenumfangsrand und einem Außenumfangsrand sowie einer Oberseite und einer Unterseite,
einen zweiten umlaufenden Ring (12) aus flexiblem Folienmaterial mit einem Innenumfangsrand und einem Außenumfangsrand, wobei der Innenumfangsrand des zweiten umlaufenden Rings am Innenumfangsrand des ersten umlaufenden Rings befestigt ist, und
ein kreisförmiges, aus flexiblem Folienmaterial (16) bestehendes Stück, bei dem ein Umfangsrand am Außenrand des zweiten umlaufenden Rings befestigt ist,
so daß eine zusammenklappbare Kammer geformt ist.

19. Behandlungssystem nach Anspruch 18, wobei die Unterseite des ersten umlaufenden Rings (14) eine klebende Fläche aufweist.

20. Behandlungssystem nach Anspruch 19, wobei das kreisförmige Stück aus flexiblem Folienmaterial (16) im wesentlichen transparent bzw. durchsichtig ist.

21. Behandlungssystem nach Anspruch 14, wobei zumindest ein Abschnitt der Kammer selbstreparierend bzw. -schließend ist.

22. Behandlungssystem nach Anspruch 14, wobei die Kammer ferner ein Zulaßmittel (28, 29, 34, 35) aufweist.

23. Behandlungssystem nach Anspruch 22, wobei das Zulaßmittel mindestens (je) einen Einlaß (28, 34) und eine(n) Auslaß(öffnung) (29, 35) umfaßt.

24. Behandlungssystem nach Anspruch 18, ferner umfassend einen neben der fluidumgefüllten Kammer angeordneten und eine Verbindung damit aufweisenden zweiten Raum, aus welchem ein Behandlungszusatz in die fluidumgefüllte Kammer freigesetzt werden kann.

25. Behandlungssystem nach Anspruch 18, wobei ferner innerhalb der Kammer ein mit einer permeablen Membran versehener Raum vorgesehen ist.

26. Behandlungssystem nach Anspruch 1, wobei der Umfang der den Umfang der Wunde kontaktierenden Fläche der Kammer ferner ein H-Typ-Verbindungs(klebe)band aufweist, das durch eine Brücke (44) verbundene parallele Ebenen bzw. Flächen (41) aus flexiblem Folienmaterial umfaßt, wobei die Außenflächen des Folienmaterials eine Klebmittelfläche (43) aufweisen.

27. Behandlungssystem nach Anspruch 1, wobei die fluidumgefüllte Kammer weiterhin ein Befestigungselement (52) zum Vereinigen von mehreren fluidgefüllten Kammern miteinander aufweist, umfassend:
einen ersten Befestigungsstreifen mit einer klebenden Seite und einer gegenüberliegenden, an einer ersten Kammer befestigten Seite,
einen zweiten Befestigungsstreifen mit einer klebenden Seite und einer gegenüberliegenden, an einer zweiten Kammer befestigten Seite und
eine Brücke mit einem Rand, der an der nichtklebenden Seite des ersten Befestigungsstreifens befestigt ist, und dem anderen Rand, der an der nichtklebenden Seite des zweiten Befestigungsstreifens befestigt ist.

28. Behandlungssystem nach Anspruch 27, wobei die Streifen und die Brücke aus einem flexiblen Werkstoff bestehen.

29. Behandlungssystem nach Anspruch 28, wobei der Brücke ein Versteifungselement zugeordnet ist.

30. Behandlungssystem nach Anspruch 28, wobei das Versteifungselement in die Brücke eingebettet ist.

31. Behandlungssystem nach Anspruch 6 für die Dialyse, wobei die Behandlungszusätze und die Steuerung der Behandlungsvariablen derart gewählt sind, daß sich ein günstiger Gradient einstellt.

32. Behandlungssystem nach Anspruch 1, ferner umfassend ein in der fluidumgefüllten Kammer enthaltenes Medikament, das der Wunde zugeführt werden soll.

33. Behandlungssystem nach Anspruch 2, wobei der Behandlungszusatz aus analogen Hautzellen besteht.

34. Behandlungssystem nach einem der Ansprüche 1 bis 33 zur Verwendung in der Medizin.

## Revendications

1. Système de traitement pour blessures, ledit système de traitement comprenant :
une chambre flexible adaptée (10, 20, 30, 40, 50) ayant un pli à soufflets (12) pour contenir un fluide de traitement dans celle-ci et pouvant être scellée autour de la périphérie d'une blessure ;
un fluide de traitement comprenant une solution saline et contenant de l'oxygène, du dioxyde de carbone et ayant un pH physiologique approprié pour guérir une blessure dans la chambre ; et
un moyen de contrôle pour évaluer l'état de la blessure, la chambre permettant le contrôle de la concentration d'oxygène, du dioxyde de carbone et du pH.

2. Système de traitement selon la revendication 1, comprenant en outre un additif de traitement choisi, en fonction de l'état de la blessure, dans le groupe constitué des anesthésiques, des antibiotiques, des produits chimiothérapeutiques, des facteurs de croissance, d'un milieu de culture cellulaire, des cellules, de l'oxygène, des agents tamponnants, des enzymes et des modulateurs immuns.

3. Système de traitement selon la revendication 1, comprenant en outre un moyen de contrôle pour des variables de traitement choisies dans le groupe constitué de la température, de la pression colloïdeosmotique, du pH, de la concentration ionique et de la teneur en oxygène.

4. Système de traitement selon la revendication 2, comprenant un fluide de traitement choisi en fonction de l'état de la blessure.

5. Système de traitement selon la revendication 4, comprenant en outre des moyens d'accès (28, 29, 34, 35) pour introduire des fluides de traitement dans la chambre et pour extraire un fluide de ladite chambre, ledit fluide de traitement étant changé périodiquement en fonction de l'état de la blessure.

6. Système de traitement selon la revendication 5, comprenant des moyens pour perfuser en continu un fluide de traitement au travers de ladite chambre.

7. Système de traitement selon la revendication 6, dans lequel ledit fluide de traitement est traité pour décontaminer ledit fluide.

8. Système de traitement selon la revendication 7, comprenant des moyens pour introduire un fluide de traitement frais dans le système.

9. Système de traitement selon la revendication 1, dans lequel ledit moyen de contrôle comprend un moyen d'inspection visuelle par lequel la blessure peut être observée visuellement et ledit fluide de traitement peut être observé visuellement.

10. Système de traitement selon la revendication 9, dans lequel ledit moyen d'inspection visuelle comprend une section transparente (16) de ladite chambre.

11. Système de traitement selon la revendication 1, dans lequel ledit moyen de contrôle comprend un moyen pour extraire du fluide (28, 29, 34, 35) du système et un moyen pour analyser ledit fluide.

12. Système de traitement selon la revendication 11, dans lequel ledit moyen pour analyser ledit fluide comprend en outre un moyen pour analyser le fluide extrait quant à la teneur en protéine ou aux microorganismes.

13. Système de traitement selon la revendication 1, dans lequel ladite chambre a un adhésif (22) sur la surface en contact avec la périphérie de la blessure.

14. Système de traitement selon la revendication 1, dans lequel la chambre comprend un matériau qui est auto-réparateur pour permettre l'injection ou l'extraction de matière au travers des parois de la chambre par une aiguille.

15. Système de traitement selon la revendication 5, dans lequel lesdits moyens d'accès comprennent au moins une ouverture d'entrée (28, 34) et une ouverture de sortie (29, 35).

16. Système de traitement selon la revendication 1, comprenant en outre un second compartiment adjacent à la chambre remplie de fluide ayant un raccord à celle-ci, à partir duquel un additif de traitement peut être libéré dans ladite chambre remplie de fluide.

17. Système de traitement selon la revendication 1, dans lequel ladite chambre comprend en outre un compartiment dans ladite chambre, ledit compartiment ayant une membrane perméable.

18. Système de traitement selon la revendication 1, dans lequel ladite chambre comprend :
un premier anneau circulaire (14) en matière de feuille flexible ayant un bord périphérique intérieur et un bord périphérique extérieur et ayant une face supérieure et une face inférieure ;
un second anneau circulaire (12) en matière de feuille flexible ayant un bord périphérique intérieur et un bord périphérique extérieur, ledit bord périphérique intérieur dudit second anneau circulaire étant fixé audit bord périphérique intérieur dudit premier anneau circulaire ; et
un morceau circulaire de matière en feuille flexible (16) ayant un bord périphérique fixé audit bord extérieur dudit second anneau circulaire ;
une chambre pouvant s'affaisser étant ainsi formée.

19. Système de traitement selon la revendication 18, dans lequel la face intérieure dudit premier anneau circulaire (14) a une surface adhésive.

20. Système de traitement selon la revendication 19, dans lequel ledit morceau circulaire de matière en feuille flexible (16) est substantiellement transparent.

21. Système de traitement selon la revendication 14, dans lequel au moins une partie de ladite chambre est auto-réparatrice.

22. Système de traitement selon la revendication 14, dans lequel ladite chambre comprend en outre des moyens d'accès (28, 29, 34, 35).

23. Système de traitement selon la revendication 22, dans lequel lesdits moyens d'accès comprennent au moins une ouverture d'entrée (28, 34) et une ouverture de sortie (29, 35).

24. Système de traitement selon la revendication 18, comprenant en outre un second compartiment adjacent à la chambre remplie de fluide ayant un raccord à celle-ci, à partir duquel un additif de traitement peut être libéré dans ladite chambre remplie de fluide.

25. Système de traitement selon la revendication 18, dans lequel ladite chambre comprend en outre un compartiment dans ladite chambre, ledit compartiment ayant une membrane perméable.

26. Système de traitement selon la revendication 1, dans lequel la périphérie de la surface de la chambre en contact avec la périphérie de la blessure comprend en outre une bande de joint de type H, ladite bande comprenant des plans parallèles (42) de matière en feuille flexible reliés par un pont (44), les surfaces extérieures de ladite matière en feuille ayant une surface adhésive (43).

27. Système de traitement selon la revendication 1, dans lequel la chambre remplie de fluide comprend en outre une fixation (52) pour joindre plus d'une chambre remplie de fluide à une autre, comprenant :
une première bande d'attachement ayant un côté adhésif et un côté opposé fixé à une première chambre ;
une seconde bande d'attachement ayant un côté adhésif et un côté opposé fixé à une seconde chambre ; et
un pont ayant un bord fixé au côté non adhésif de ladite première bande d'attachement et l'autre bord fixé au côté non adhésif de ladite seconde bande d'attachement.

28. Système de traitement selon la revendication 27, dans lequel lesdites bandes et ledit pont sont une matière flexible.

29. Système de traitement selon la revendication 28, dans lequel ledit pont est associé à un élément renfort.

30. Système de traitement selon la revendication 28, dans lequel ledit élément renfort est inclus dans ledit pont.

31. Système de traitement selon la revendication 6 pour dialyse, dans lequel lesdits additifs de traitement et le contrôle desdites variables de traitement sont choisis pour créer un gradient favorable.

32. Système de traitement selon la revendication 1, comprenant en outre, dans la chambre remplie de fluide, un médicament à délivrer à la blessure.

33. Système de traitement selon la revendication 2, dans lequel ledit additif de traitement est des cellules analogues de peau.

34. Système de traitement selon l'une quelconque des revendications 1 à 33, pour l'utilisation en médecine.
